(19) Europäisches Patentamt
European Patent Office
Office européen des brevets

(11) **EP 1 477 807 B1**

(12) **EUROPÄISCHE PATENTSCHRIFT**

(45) Veröffentlichungstag und Bekanntmachung des Hinweises auf die Patenterteilung:
**15.12.2010 Patentblatt 2010/50**

(21) Anmeldenummer: **04010657.7**

(22) Anmeldetag: **05.05.2004**

(51) Int Cl.:
**G01N 33/50** *(2006.01)*

(54) **Verfahren zur Bestimmung des Vorhandenseins von Thrombozyteninhibitoren in einer Blutprobe**

Method for determining the presence of inhibitors of thrombocytes in a blood sample

Méthode pour déterminer la présence d'inhibiteurs de thrombocyte dans un échantillon sanguin.

(84) Benannte Vertragsstaaten:
**AT BE BG CH CY CZ DE DK EE ES FI FR GB GR HU IE IT LI LU MC NL PL PT RO SE SI SK TR**

(30) Priorität: **12.05.2003 DE 10321431**

(43) Veröffentlichungstag der Anmeldung:
**17.11.2004 Patentblatt 2004/47**

(73) Patentinhaber: **Pachmann, Ulrich**
**95448 Bayreuth (DE)**

(72) Erfinder: **Pachmann, Ulrich**
**95448 Bayreuth (DE)**

(74) Vertreter: **Gassner, Wolfgang**
**Dr. Gassner & Partner**
**Patentanwälte**
**Marie-Curie-Strasse 1**
**91052 Erlangen (DE)**

(56) Entgegenhaltungen:
**WO-A-96/10749**

- **FRELINGER A L ET AL: "NOVEL METHODS FOR ASSESSING PLATELET FUNCTION" AMERICAN HEART JOURNAL, MOSBY- YEAR BOOK INC., ST. LOUIS, MO, US, Bd. 136, Nr. SUPPL, Mai 1998 (1998-05), Seiten S184-S186, XP001119048 ISSN: 0002-8703**
- **B. JILMA: "Platelet function analyzer (PFA-100): A tool to quantify congenital or acquired platelet dysfunction" JOURNAL OF LABORATORY AND CLINICAL MEDICINE, Bd. 138, Nr. 3, Dezember 2001 (2001-12), XP002467274**
- **NICHOLSON ET AL: "Assessment of platelet function assays" AMERICAN HEART JOURNAL, MOSBY- YEAR BOOK INC., ST. LOUIS, MO, US, Bd. 135, Nr. 5, Mai 1998 (1998-05), Seiten S170-S178, XP005179032 ISSN: 0002-8703**
- **MADAN MINA ET AL: "Rapid assessment of glycoprotein IIb/IIIa blockade with the platelet function analyzer (PFA-100) during percutaneous coronary intervention" AMERICAN HEART JOURNAL, Bd. 141, Nr. 2, Februar 2001 (2001-02), Seiten 226-233, XP002467275 ISSN: 0002-8703**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Bestimmung des Vorhandenseins von Thrombozyteninhibitoren in einer Blutprobe.

Im Stand der Technik ist es bekannt, die primäre Hämostase-Funktion von Thrombozyten anhand der Blutungszeit zu bestimmen. Dabei wird die Nachblutungsdauer nach einer definierten Stichinzision gemessen. Das Verfahren ist sehr ungenau und zeitaufwändig. Bei Thrombozyten-Funktionsstörungen kann es zu Nachblutungskomplikationen kommen. Für die Bestimmung von Thrombozyten-Funktionsinhibitoren, z. B. inhibitorischen Thrombozyten-Autoantikörpern, ist das bekannte Messverfahren ungeeignet. Ein dazu geeignetes Verfahren steht bisher nicht zur Verfügung.

Die Thrombozytenfunktion kann auch automatisiert durch Messung der in-vitro Blutungszeit gemessen werden. Das kann beispielsweise mit dem Gerät PFA-100® der Firma Dade-Behring durchgeführt werden. Dabei wird pH-gepuffertes mittels Citrat gerinnungsinhibiertes Vollblut mittels einer Vakuumpumpe aus einem Reservoir durch eine Kapillare und die Öffnung einer Membran gefördert, wodurch hohe Scherkräfte entstehen. Die Membran ist mit Kollagen beschichtet und enthält Epinephrin oder Andenosindiphosphat. Beim Durchströmen der Kapillare und der Membranöffnung werden Thrombozyten aktiviert und führen durch Aggregation zum allmählichen Verschluss der Membranöffnung. Der Verschluss der Membranöffnung wird über im Vakuumsystem vorhandene Drucksensoren detektiert. Als Messwert wird dann die Zeit zwischen Beginn der Messung und dem vollständigen Verschluss der Membranöffnung als so genannte Verschlusszeit ausgegeben (vgl. dazu Madan et al., American Heart Journal, Bd. 141, Nr. 2, (2001), 226-233).

Aufgabe der Erfindung ist die Bereitstellung eines Verfahrens zur Bestimmung des Vorhandenseins von Thrombozyteninhibitoren in einer Blutprobe einer ersten Person.

Diese Aufgabe wird durch ein Verfahren mit den Merkmalen des Patentanspruchs 1 gelöst. Zweckmäßige Ausgestaltungen ergeben sich aus den Merkmalen der Ansprüche 2 bis 10.

Bevorzugt wird das Verfahren mit Hilfe des Thromb,ozyten-Funktions-Analysators PFA-100® der Firma Dade-Behring durchgeführt. Ein Teil des thrombozytenfreien Plasmas des Vollbluts einer zweiten oder weiteren Person, z.B. eines gesunden Probanden, wird durch thrombozytenfreies Plasma des Vollbluts der ersten Person ersetzt, welches möglicherweise Thrombozyteninhibitoren enthält. Durch anschließendes Vermischen des Plasmas mit den sonstigen Blutbestandteilen wird die Probe zu einer gemischten Probe teilrekonstituiert. Als Normalwert dient die Verschlusszeit einer ebenfalls zentrifugierten Vollblutprobe eines gesunden Probanden, welche mit Plasma aus derselben Vollblutprobe des gesunden Probanden rekonstituiert oder mit Plasma eines weiteren gesunden Probanden teilrekonstituiert worden ist. Unter Rekonstituieren wird das Vermischen von Blutbestandteilen verstanden, die ursprünglich aus derselben Vollblutprobe stammen und von dieser abgetrennt worden sind. Durch das Vermischen entsteht eine in der Zusammensetzung der Vollblutprobe entsprechende Probe. Unter Teilrekonstituieren wird das Vermischen von Blutbestandteilen verstanden, die ursprünglich aus verschiedenen Vollblutproben stammen und von diesen abgetrennt worden sind.

Die erste und die zweite Vollblutprobe sind bei dem Verfahren so zu wählen, dass die Isoagglutinine der ersten Vollblutprobe nicht die Erythrozyten der zweiten Vollblutprobe agglutinieren können. Die Auswahl geeigneter Blutproben kann gemäß der folgenden Tabelle erfolgen:

| Blutgruppe der zweiten Vollblutprobe | erste Vollblutprobe Blutgruppe A | erste Vollblutprobe Blutgruppe B | erste Vollblutprobe Blutgruppe 0 | erste Vollblutprobe Blutgruppe AB |
|---|---|---|---|---|
| A | + | - | - | + |
| B | - | + | - | + |
| 0 | + | + | + | + |
| AB | - | - | - | + |
| "+" bedeutet dabei, dass die Isoagglutinine der ersten Vollblutprobe nicht die Erythrozyten der zweiten Vollblutprobe agglutinieren können. Die mit "+" gezeichneten Kombinationen der ersten und zweiten Vollblutprobe sind somit kompatibel und können für das erfindungsgemäße Verfahren eingesetzt werden. | | | | |

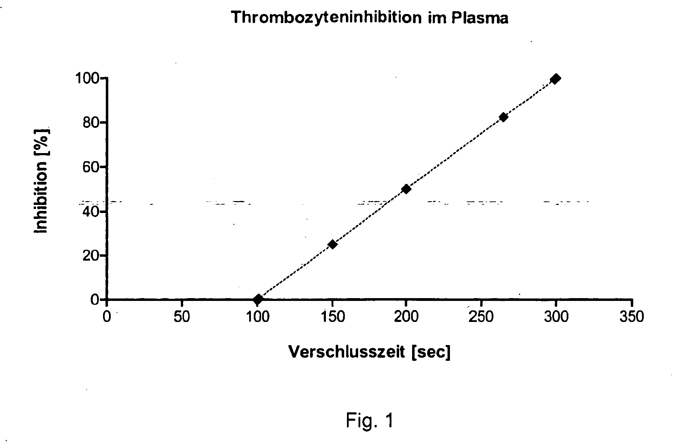

Das Verfahren ermöglicht die Feststellung einer normierten prozentualen Inhibition im Verhältnis zum Normalwert. Die prozentuale Inhibition kann beispielsweise aus der in Fig. 1 dargestellten Grafik abgelesen werden, in der eine Verschlusszeit von 100 Sekunden (s) dem Normalwert entspricht. Ein erst am oder nicht bis zum Ende der maximalen Messzeit, hier 300 s, erfolgender Verschluss entspricht einer Inhibition von maximal 100 %.

Alternativ kann die prozentuale Inhibition auch nach folgender Formel berechnet werden, wobei MZ für Messzeit, VZ für Verschlusszeit und VB für Vollblutprobe steht:

$$Inhibition[\%]=100(1-\frac{\text{maximale MZ [s] – VZ der gemischten Probe [s]}}{\text{maximale MZ [s] – VZ der zweiten oder weiteren VB [s]}})$$

Das vorgeschlagene Verfahren weist die folgenden Vorteile auf:

a) Es können Thrombozyteninhibitoren in Patienten bestimmt werden, die eine Blutungsneigung ohne eine extreme Thrombozytopenie aufweisen.

b) Es kann geprüft werden, ob Fremd-Thrombozyten-Konzentrate funktionell inhibiert werden und ob sie somit zur Therapie geeignet sind oder nicht.

c) Es kann untersucht werden, ob die Thrombozyten-Funktion durch Medikamente beeinflusst wird.

Nachfolgend wird die Erfindung anhand eines Ausführungsbeispiels näher erläutert.

Um zu prüfen, ob das Plasma einer ersten Vollblutprobe inhibitorische Thrombozyten-Antikörper enthält wird wie folgt vorgegangen:

Je eine auf pH 5,5 gepufferte und mittels 129 mmol/l Natriumcitrat gerinnungsinhibierte erste Vollblutprobe eines Patienten sowie eine zweite und dritte Vollblutprobe von einer Kontrollperson oder von zwei voneinander verschiedenen Kontrollpersonen der Blutgruppe 0 mit einem Volumen von jeweils 3,5 ml werden spätestens 4 Stunden nach deren Entnahme für 5 min bei 10 000 g zentrifugiert. In den Proben bildet sich dadurch ein Überstand aus im Wesentlichen plättchenfreiem Plasma. Von der dritten Vollblutprobe werden 840 $\mu$l des Plasmas entnommen und verworfen und durch 840 $\mu$l des Plasmas der zweiten Vollblutprobe ersetzt. Die von der zweiten Vollblutprobe entnommenen 840 $\mu$l Plasma werden durch 840 $\mu$l des Plasmas der ersten Vollblutprobe ersetzt.

Die teilweise Plasma der zweiten Vollblutprobe enthaltende dritte Vollblutprobe und die teilweise Plasma der ersten Vollblutprobe enthaltende zweite Vollblutprobe werden für 2 min auf einem Rollenmischer gründlich gemischt und dadurch teilrekonstituiert. Die Verschlusszeit der beiden Proben werden im PFA-100® bestimmt. Durch ins Verhältnis setzen der für die teilrekonstruierte zweite Vollblutprobe bestimmten Verschlusszeit zu der für die teilrekonstruierte dritte Vollblutprobe als Normwert ermittelten Verschlusszeit wird die prozentuale Inhibition gemäß obiger Formel bestimmt. Alternativ kann statt einer teilrekonstituierten dritten Vollblutprobe auch eine nur mit Plasma dieser dritten Vollblutprobe rekonstituierte Probe oder direkt die nur gerinnungsinhibierte dritte Vollblutprobe eingesetzt werden.

**Patentansprüche**

1. Verfahren zur Bestimmung des Vorhandenseins von Thrombozyteninhibitoren in einer ersten Vollblutprobe einer ersten Person mit folgenden Schritten:

a) Bereitstellen der ersten gerinnungsinhibierten Vollblutprobe,
b) Bereitstellen einer gerinnungsinhibierten zweiten Vollblutprobe einer zweiten Person, die so gewählt ist, dass die Isoagglutinine der ersten Vollblutprobe nicht die Erythrozyten der zweiten Vollblutprobe agglutinieren können,
c) Zentrifugieren der ersten und der zweiten Vollblutprobe, so dass sich als Überstand ein Plasma bildet, welches im Wesentlichen frei von Blutplättchen ist,
d) Entnahme einer definierten Menge des Plasmas der zweiten Vollblutprobe und Ersetzen dieses Plasmas durch Zugabe von aus der ersten Vollblutprobe entnommenem Plasma,
e) Vermischen der Blutbestandteile der so gebildeten gemischten Probe,
f) Messen der Zeit für eine durch Thrombozyten ausgelöste Gerinnung dieser gemischten Probe und ins Verhältnis setzen dieser Zeit zu.der Zeit für eine durch Thrombozyten ausgelöste Gerinnung einer gerinnungsinhibierten dritten Vollblutprobe der zweiten oder einer anderen Person.

2. Verfahren nach Anspruch 1, wobei die erste, zweite und/oder dritte Vollblutprobe mittels Citrat gerinnungsinhibiert ist.

3. Verfahren nach Anspruch 1 oder 2, wobei die erste, zweite und/oder dritte Vollblutprobe pH-gepuffert ist.

4. Verfahren nach Anspruch 3, wobei die erste, zweite und/oder dritte Vollblutprobe auf einen pH-Wert von etwa 5,5 gepuffert ist.

**5.** Verfahren nach einem der vorhergehenden Ansprüche, wobei die Konzentration an Citrat in der ersten, zweiten und/oder dritten Vollblutprobe etwa 129 mmol/l beträgt.

**6.** Verfahren nach einem der vorhergehenden Ansprüche, wobei die gerinnungsinhibierte dritte Vollblutprobe rekonstituiert oder teilrekonstituiert ist.

**7.** Verfahren nach einem der vorhergehenden Ansprüche, wobei beim Schritt d) die Menge des zum Ersatz zugesetzten von der ersten Vollblutprobe entnommenen Plasmas im Wesentlichen identisch ist mit der aus der zweiten Vollblutprobe entnommenen definierten Menge des Plasmas.

**8.** Verfahren nach einem der vorhergehenden Ansprüche, wobei die Menge des bei Schritt lit. d von der ersten und/oder zweiten Vollblutprobe entnommenen Plasmas etwa 24 % des Gesamtvolumens dieser Vollblutprobe entspricht.

**9.** Verfahren nach einem der vorhergehenden Ansprüche, wobei beim Schritt. f) die Zeit für die Gerinnung mittels eines automatischen Plättchen-Funktions-Analysators, insbesondere mittels des PFA-100® der Firma Dade-Behring, gemessen wird.

**10.** Verfahren nach einem der vorhergehenden Ansprüche, wobei beim Schritt f) zum Bewirken der durch Thrombozyten ausgelösten Gerinnung der gemischten Probe und der zweiten oder der anderen Vollblutprobe Kollagen und Adenosindiphosphat oder Kollagen und Epinephrin eingesetzt werden.


**Claims**

**1.** Method for determining the presence of thrombocyte inhibitors in a first whole blood sample of a first person, comprising the following steps:

a) providing the first coagulation-inhibited whole blood sample,
b) providing a coagulation-inhibited second whole blood sample of a second person that is chosen such that the isoagglutinins of the first whole blood sample cannot agglutinate the erythrocytes of the second whole blood sample,
c) centrifuging the first and the second whole blood sample to yield a supernatant comprising plasma which is substantially free of blood platelets,
d) removing a defined amount of the plasma of the second whole blood sample and replacing this plasma by adding plasma removed from the first whole blood sample,
e) mixing the blood constituents of the mixed sample thus formed,
f) measuring the time taken for a thrombocyte-triggered coagulation of this mixed sample and relating this time to the time taken for a thrombocyte-triggered coagulation of a coagulation-inhibited third whole blood sample of the second person or another person.

**2.** Method according to Claim 1, wherein the first, second and/or third whole blood sample is coagulation-inhibited by means of citrate.

**3.** Method according to Claim 1 or 2, wherein the first, second and/or third whole blood sample is pH-buffered.

**4.** Method according to Claim 3, wherein the first, second and/or third whole blood sample is buffered at a pH of about 5.5.

**5.** Method according to any one of the preceding claims, wherein the concentration of citrate in the first, second and/or third whole blood sample is about 129 mmol/l.

**6.** Method according to any one of the preceding claims, wherein the coagulation-inhibited third whole blood sample is reconstituted or partially reconstituted.

**7.** Method according to any one of the preceding claims, wherein, in step d), the amount of the plasma removed from the first whole blood sample and added as a replacement is essentially identical to the defined amount of the plasma removed from the second whole blood sample.

**8.** Method according to any one of the preceding claims, wherein the amount of the plasma removed from the first and/or second whole blood sample in step d) corresponds to about 24 % of the total volume of this whole blood sample.

9. Method according to any one of the preceding claims, wherein, in step f), the time taken for the coagulation is measured by means of an automatic platelet-function analyser, more particularly by means of the PFA-100® from Dade-Behring.

10. Method according to any one of the preceding claims, wherein, in step f), thrombocyte-triggered coagulation of the mixed sample and of the second whole blood sample or the other whole blood sample is effected by using collagen and adenosine diphosphate or collagen and adrenaline.


**Revendications**

1. Procédé de détermination de la présence d'inhibiteurs de thrombocytes dans un premier échantillon de sang total d'une première personne, comprenant les étapes suivantes :

a) mise à disposition du premier échantillon de sang total avec inhibition de la coagulation,
b) mise à disposition d'un second échantillon de sang total, avec inhibition de la coagulation, d'une seconde personne, qui est sélectionné de façon à ce que les isoagglutinines du premier échantillon de sang total ne puissent pas agglutiner les hématies du second échantillon de sang total,
c) centrifugation du premier et du second échantillons de sang total, de façon à ce que se forme en tant que surnageant un plasma qui est essentiellement exempt de plaquettes,
d) prélèvement d'une quantité définie du plasma du second échantillon de sang total et remplacement de ce plasma par ajout de plasma prélevé du premier échantillon de sang total,
e) mélange des constituants sanguins de l'échantillon mixte ainsi formé,
f) mesure du temps de coagulation, déclenché par des thrombocytes, de cet échantillon mixte et mise en relation de ce temps avec le temps de coagulation, déclenché par des thrombocytes, d'un troisième échantillon de sang total, avec inhibition de la coagulation, de la seconde ou d'une autre personne.

2. Procédé selon la revendication 1, dans lequel le premier, le second et/ou le troisième échantillons de sang total ont une coagulation inhibée par du citrate.

3. Procédé selon la revendication 1 ou 2, dans lequel le premier, le second et/ou le troisième échantillons de sang total ont un pH tamponné.

4. Procédé selon la revendication 3, dans lequel le premier, le second et/ou le troisième échantillons de sang total sont tamponnés à une valeur de pH environ égale à 5,5.

5. Procédé selon l'une des revendications précédentes, dans lequel la concentration en citrate dans les premier, second et/ou troisième échantillons de sang total est environ égale à 129 mmol/l.

6. Procédé selon l'une des revendications précédentes, dans lequel le troisième échantillon de sang total avec coagulation inhibée est reconstitué ou partiellement reconstitué.

7. Procédé selon l'une des revendications précédentes, dans lequel à l'étape d), la quantité du plasma prélevé du premier échantillon de sang total et ajouté en remplacement est essentiellement identique à la quantité du plasma définie prélevée du second échantillon de sang total.

8. Procédé selon l'une des revendications précédentes, dans lequel la quantité du plasma prélevé à l'étape d) du premier et/ou du second échantillon de sang total correspond à environ 24 % du volume total de cet échantillon de sang total.

9. Procédé selon l'une des revendications précédentes, dans lequel à l'étape f), le temps de coagulation est mesuré au moyen d'un analyseur automatique de la fonction plaquettaire, en particulier au moyen de l'appareil PFA-100® de la société Dade-Behring.

10. Procédé selon l'une des revendications précédentes, dans lequel à l'étape f), pour provoquer la coagulation, déclenchée par les thrombocytes, de l'échantillon mixte et du second ou de l'autre échantillon de sang total, on utilise du collagène et de l'adénosine diphosphate ou du collagène et de l'épinéphrine.

**Thrombozyteninhibition im Plasma**

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **Madan et al.** *American Heart Journal,* 2001, vol. 141 (2), 226-233